# EUROPEAN PATENT APPLICATION

(11) **EP 4 098 754 A1**
(43) Date of publication of application: **07.12.2022**
(21) Application number: 21305757.3
(22) Date of filing: 04.06.2021
(51) Int. Cl.: C12Q 1/6886

(54) **RNA SIGNATURE OF PDAC SENSITIVITIES TO ANTICANCER TREATMENTS**

(71) Applicant: INSTITUT NATIONAL DE LA SANTE ET DE LA RECHERCHE MEDICALE - INSERM, 75013 Paris (FR); Centre national de la recherche scientifique, 75016 Paris (FR); Université d'Aix-Marseille, 13007 Marseille (FR); Institut Paoli Calmettes, 13009 Marseille (FR)
(72) Inventor: IOVANNA, Juan, 13008 MARSEILLE (FR); DUSETTI, Nelson, 13008 MARSEILLE (FR); FRAUNHOFFER, Nicolas, 13006 MARSEILLE (FR)
(74) Representative: Lavoix

(57) **Abstract**

The present invention relates to a method and a kit for determining if a patient having pancreatic ductal adenocarcinoma (PDAC) is sensitive to a treatment. It further relates to a method for determining a suitable treatment for a patient having PDAC.

## Description

The present invention relates to a method and a kit for determining if a patient having pancreatic ductal adenocarcinoma (PDAC) is sensitive to a treatment. It further relates to a method for determining a suitable treatment for a patient having PDAC.

Pancreatic ductal adenocarcinoma (PDAC) is the fourth leading cause of cancer-related mortality in western countries and bears one of the poorest prognoses with a five-year survival rate of 6%. In addition to frequent late-stage diagnosis, this dismal outcome can also be explained by the lack of effective therapies. Targeted therapies and immunotherapies have failed to improve unselected patients' outcomes, making chemotherapy the only effective systemic treatment. Despite being a generally refractory cancer, it has been shown that the molecularly guided selection of small patient subgroups increased the efficacy for some therapies such as Olaparib for patients with germline BRCA mutations (approximately 5% of patients) or immunotherapy for mismatch-repair deficient tumors (approximately 1% of patients). These studies demonstrate the advantage of molecular stratification for therapeutic decisions and introduce an incremental model for PDAC, solving one subgroup at a time.

Today, the selection of systemic therapy is based on patient's fitness rather than on the potential efficacy of a particular chemotherapeutic regimen.

Companion diagnostics are used to guide the choice of therapy by selecting patients that have a higher chance of responding to a given therapeutic agent. With an ever-growing number of regimens composed of multiple chemotherapies, companion diagnostics has not only the potential to improve survival by matching drugs to likely responders, but also to reduce adverse effects by avoiding unnecessary highly toxic regimens.

For example, FOLFIRINOX is one of the main chemotherapy treatments for pancreatic ductal adenocarcinoma. FOLFIRINOX is the combination of three different chemotherapy drugs and a vitamin: Fluoro-uracil (5-FU), Irinotecan, and Oxaliplatin. It also includes folinic acid (Leucovorin). However, only one third of patients treated with FOLFIRINOX respond to the treatment. The remaining two third suffer of the side effects of FOLFIRINOX without any benefice.

Another example is Gemcitabine, it remains to date the most effective monotherapy in PDAC but with an estimated response rate of 10% to 23% in advanced patients.

There is a need for a new method for determining the best treatment for patient having pancreatic ductal adenocarcinoma, and thus avoiding them unnecessary treatments, or unnecessary strong side-effects.

The inventors discovered that RNA signature of PDAC cells can be used as a marker for determining sensitivity to a treatment of a patient having pancreatic ductal adenocarcinoma (PDAC).

### Summary of the invention

In a first aspect, the present invention provides a method, preferably an *ex vivo* method, for determining if a patient having pancreatic ductal adenocarcinoma (PDAC) is sensitive to a treatment, comprising:
a) detecting total RNA transcripts of cells of the PDAC of said patient; and
b) determining the patient as sensitive to said treatment, if the RNA signature of PDAC cells of said patient corresponds to the sensitivity RNA signature of said treatment,
wherein the sensitivity RNA signature of a treatment is a set of RNA transcripts which presence or absence is significantly correlated to sensitivity to said treatment.

The invention also concerns gemcitabine, oxaliplatin, 5 fluoro-uracil (5-FU), irinotecan, a taxane or a combination thereof, for use for treating PDAC in a patient, wherein said patient having a PDAC has been determined sensitive to gemcitabine, oxaliplatin, 5 fluoro-uracil (5-FU), irinotecan, a taxane or a combination thereof.

In a further aspect, the invention provides a kit for determining if a patient having pancreatic ductal adenocarcinoma (PDAC) is sensitive to a treatment with gemcitabine, oxaliplatin, 5 fluoro-uracil (5-FU), irinotecan, a taxane or a combination thereof, comprising means for detecting RNA.

The present invention also relates to a method, preferably an *ex vivo* method for determining a suitable treatment for a patient having pancreatic ductal adenocarcinoma (PDAC), comprising:
a) determining if the patient is sensitive to a treatment using according to the method of the invention, and
b) deducing a suitable treatment for the patient.

### Detailed description of the invention

### Method for determining if a patient having PDAC is sensitive to a treatment

The present invention provides a method, preferably an *ex vivo* method, for determining if a patient having pancreatic ductal adenocarcinoma (PDAC) is sensitive to a treatment, comprising:
a) detecting total RNA transcripts of cells of the PDAC of said patient; and
b) determining the patient as sensitive to said treatment, if the RNA signature of PDAC cells of said patient corresponds to the sensitivity RNA signature of said treatment,
wherein the sensitivity RNA signature of a treatment is a set of RNA transcripts which presence or absence is significantly correlated to sensitivity to said treatment.

As used herein the term "patient" refers to a mammalian such as a rodent, a feline, an equine, a bovine, an ovine, a canine or a primate, and is preferably a primate and more preferably a human. The patient has been diagnosed as having PDAC.

By "PDAC" or "pancreatic ductal adenocarcinoma", it is meant a pancreatic cancer originating in the ductal epithelium of the pancreas.

"Therapy" or "treatment" includes reducing, alleviating, inhibiting, or eliminating the causes of a disease or pathological conditions, as well as treatment intended to reduce, alleviate, inhibit or eliminate symptoms of said disease or pathological condition. In particular herein these terms includes reducing the tumor size, slowing the tumor growth, eliminating the tumor, and/or inhibiting the apparition of metastasis.

By "a patient sensitive to a treatment", it is meant that the patient has a PDAC cancer on which the treatment has a positive benefice/risk balance, i.e. the treatment is able to reduce, alleviate, inhibit or eliminate symptoms of cancer. Preferably, the patient shows complete response (CR), partial response (PR), or at least is stable (stable disease SD) with said treatment.

Similarly, the PDAC is sensitive to a treatment, when the treatment is able to reduce the tumor size, slow the tumor growth, eliminate the tumor, and/or inhibit the apparition of metastasis.

The term "cells of the PDAC" or "PDAC cells" refers to at least one cell extracted from the PDAC tumor of a patient. These cells can be alive or lysed. These cells can be obtained by several techniques such as fine needle aspiration, or other type of biopsy, or following resection of a part of the tumor. They can be processed (such as purification, fractionation, enzymatic processing, freezing etc...) prior to the detection of total RNA transcripts.

RNA stands for ribonucleic acid. RNA transcripts are produced by RNA polymerase during transcription.

As used herein the term "total RNA transcripts", is all RNA transcripts that can be detected in a cell or a group of cells, by methods well-known to the skilled in the art such as RNA-seq. Preferably, detection of the total RNA transcript according to the invention is done by DNA microarray, RNA-seq, nanostring or RT-PCR.

The detection of total RNA transcripts of PDAC cells allows obtaining a full RNA signature of said cells, named RNA signature.

By "sensitivity RNA signature of a treatment" or in short "sensitivity signature", it is meant a set of RNA transcripts which presence or absence is significantly correlated to sensitivity of PDAC cells to said treatment. Preferably, the sensitivity RNA signature of a treatment comprises several RNA transcripts and the information on the type of correlation, i.e. if their presence or their absence is significantly correlated to sensitivity. Examples of such of signatures are provided in Tables 1-5.

Preferably, the sensitivity RNA signature of a treatment does not comprise information on all RNA transcript of the cells or on all transcripts existing in the patients' specie, but only RNA transcript which presence or absence have a significant correlation with the sensitivity to said treatment. Preferably, the sensitivity RNA signature of a treatment comprises all RNA transcript which presence or absence have a significant correlation with the sensitivity to said treatment.

In an embodiment of the invention, a RNA signature of PDAC cells corresponds to a sensitivity RNA signature of a treatment when all RNA transcripts of the sensitivity signature whose presence is positively correlated to sensitivity to the treatment are detected in PDAC cells, and all RNA transcript whose absence is positively correlated to sensitivity to the treatment is not detected in PDAC cells.

In another embodiment, correspondence between RNA signature of PDAC cells and a sensitivity RNA signature of a treatment can be evaluated by a score. The algorithm calculating the score can be statistically determined by cohort studies. The more the signature of PDAC cells is close to the sensitivity RNA signature of a treatment, the higher is the score.

When the score is higher than a predetermined value (hereby threshold), the patient is determined as being sensitive to the treatment. Said threshold can be defined by various methods such as a mean value observed in a population or cohort, or by ROC analysis. According to the invention, the threshold may be determined by a plurality of samples, preferably more than 5, 50, 100, 200 or 500 samples.

Such score can also be used for stratification of patient population. For example, in one embodiment of the invention, the quartile of the population with the lowest score is considered not sensitive to the treatment, the quartile of the population with the highest score is considered highly sensitive to the treatment, and the rest of the population is considered as normally sensitive.

In an embodiment the method for determining if a patient having pancreatic ductal adenocarcinoma (PDAC) is sensitive to a treatment according to the invention, is for determining if the patient is sensitive to a treatment with gemcitabine, oxaliplatin, 5 fluoro-uracil (5-FU), irinotecan, a taxane or a combination thereof, wherein :
a) the patient is determined as being sensitive to Gemcitabine, if the RNA signature of PDAC cells corresponds to Table 1, and/or
b) the patient is determined as being sensitive to Oxaliplatin, if the RNA signature of PDAC cells corresponds to Table 2, and /or
c) the patient is determined as being sensitive to 5-FU, if the RNA signature of PDAC cells corresponds to Table 3, and/or
d) the patient is determined as being sensitive to Irinotecan, if the RNA signature of PDAC cells corresponds to Table 4, and/or
e) the patient is determined as being sensitive to taxane, if the RNA signature of PDAC cells corresponds to Table 5.

Gemcitabine (ATCC: L01BC05, 2', 2'-difluoro 2'deoxycytidine) is used to treat a number of types of cancer including pancreatic cancer. It is part of the nucleoside analog family of medication and block the creation of new DNA, which results in cell death.

The inventors determined that the sensitivity RNA signature of Gemcitabine is as described in Table 1.

**Table 1: sensitivity RNA signature of Gemcitabine**

| **Gene** | **Correlation** |
|---|---|
| MDK | Negative |
| MTAP | Negative |
| UCHL1 | Negative |
| CALB2 | Negative |
| FLNC | Negative |
| NES | Negative |
| SMAD4 | Negative |
| NIBAN1 | Negative |
| HKDC1 | Negative |
| PLAU | Negative |
| QPRT | Negative |
| OLFML2A | Negative |
| ANGPTL4 | Positive |
| BICDL1 | Positive |
| EGR3 | Positive |
| MUC1 | Positive |
| MPDZ | Positive |
| DSP | Positive |
| FGD4 | Positive |
| INHA | Positive |
| SLC16A2 | Positive |
| RBP4 | Positive |
| SGPP2 | Positive |
| LIMCH1 | Positive |
| SCNN1A | Positive |
| TCIM | Positive |
| NRCAM | Positive |
| SERPINA3 | Positive |
| VGLL3 | Positive |
| SNAI2 | Positive |
| RAB34 | Positive |
| KRT17 | Positive |
| UGT1A8 | Positive |
| UGT1A1 | Positive |
| IRX3 | Positive |
| SERPINA5 | Positive |
| MSN | Positive |
| ROS1 | Positive |
| BASP1 | Positive |
| ALDH1A2 | Positive |
| SCNN1B | Positive |
| UGT1A3 | Positive |
| UGT1A9 | Positive |
| CGA | Positive |
| RERG | Positive |
| P3H2 | Positive |
| PCDH7 | Positive |
| F3 | Positive |
| SLC16A7 | Positive |
| UGT1A7 | Positive |
| MAP2 | Positive |
| CPM | Positive |
| PBX1 | Positive |
| UGT1A6 | Positive |
| CSGALNACT1 | Positive |
| SLPI | Positive |
| GSTM1 | Positive |
| MAOB | Positive |
| EHF | Positive |
| UGT1A10 | Positive |
| AKR1C3 | Positive |
| MTUS1 | Positive |
| KRT6A | Positive |
| NNMT | Positive |
| DNER | Positive |
| MT1X | Positive |
| LDHB | Positive |
| FKBP5 | Positive |
| PTPRM | Positive |
| PMEPA1 | Positive |
| CFH | Positive |
| PID1 | Positive |
| EREG | Positive |
| THBS1 | Positive |
| PLCXD3 | Positive |
| S100A9 | Positive |
| METTL7A | Positive |
| CCL20 | Positive |
| PGC | Positive |
| COL4A6 | Positive |
| SLC16A14 | Positive |
| SGK1 | Positive |
| BICC1 | Positive |
| RIPOR2 | Positive |
| FLRT3 | Positive |
| C3 | Positive |
| TNS4 | Positive |
| GLUL | Positive |
| FN1 | Positive |
| ALB | Positive |
| SCNN1G | Positive |
| MAOA | Positive |
| AKR1C1 | Positive |
| IL1RL1 | Positive |
| TFCP2L1 | Positive |
| SRGN | Positive |
| AKR1C2 | Positive |
| COL12A1 | Positive |
| CPE | Positive |

In this table, when the correlation column indicates "Positive", thus presence of the corresponding RNA transcript is significantly correlated to sensitivity of cells to Gemcitabine. On the opposite, when the correlation column indicates "Negative", thus absence of the corresponding RNA transcript is significantly correlated to sensitivity of cells to Gemcitabine.

Oxaliplatin (ATCC: L01XA03, (R,R)-1,2-diaminocyclohexan (ethanedioate-O,O)platin) is used to treat several cancer. It is part of the platinum-based antineoplastic family of medications, and is believed to work by blocking the duplication of DNA.

The inventors determined that the sensitivity RNA signature of Oxaliplatin is as described in Table 2.

**Table 2: Sensitivity RNA signature of Oxaliplatin**

| **Gene** | **Correlation** |
|---|---|
| SERPINB2 | Negative |
| COL12A1 | Negative |
| PTPRG | Negative |
| SLC1A3 | Negative |
| VIPR1 | Negative |
| ZNF334 | Negative |
| MCOLN3 | Negative |
| CNNM1 | Negative |
| ARFGEF3 | Negative |
| SCD5 | Negative |
| GSTM3 | Negative |
| ADAMTS9 | Negative |
| GDA | Negative |
| GATM | Negative |
| GALNT14 | Negative |
| GALNT18 | Negative |
| TLE4 | Negative |
| ERAP2 | Negative |
| PAX6 | Negative |
| DUXAP10 | Negative |
| COL4A4 | Negative |
| ZNF736 | Negative |
| NCR3LG1 | Negative |
| MT1E | Negative |
| LARGE2 | Negative |
| HES2 | Negative |
| UST | Negative |
| SLC2A3 | Negative |
| NIBAN1 | Negative |
| BACE2 | Negative |
| BDKRB2 | Negative |
| AADAC | Negative |
| SPINK5 | Negative |
| COL4A3 | Negative |
| ABCA3 | Negative |
| ICAM1 | Negative |
| SEL1L3 | Negative |
| PDE4C | Negative |
| PCSK9 | Negative |
| VWA5A | Negative |
| COLCA2 | Negative |
| CCND2 | Negative |
| ZNF618 | Negative |
| HPDL | Negative |
| VLDLR | Negative |
| CELSR3 | Negative |
| MAPRE2 | Negative |
| ESPN | Negative |
| AKT3 | Negative |
| KRT5 | Negative |
| SLC38A5 | Negative |
| P2RY1 | Negative |
| CHST4 | Negative |
| MAN1A1 | Negative |
| KCTD15 | Negative |
| CTH | Negative |
| NLRP1 | Negative |
| ANKRD1 | Negative |
| SLAIN1 | Negative |
| CLU | Negative |
| TBC1D30 | Negative |
| LYPD6B | Negative |
| PARD3B | Negative |
| PPBP | Negative |
| NRARP | Negative |
| SERPINB7 | Negative |
| COLCA1 | Negative |
| SYNE1 | Negative |
| LYPD2 | Negative |
| GPRC5B | Negative |
| JPH2 | Negative |
| CYP2C9 | Negative |
| CYP2C18 | Negative |
| ADGRL2 | Negative |
| SCIN | Negative |
| PTPN13 | Negative |
| FGFR4 | Negative |
| SLC27A2 | Negative |
| KRT13 | Negative |
| TNFAIP2 | Negative |
| DMKN | Negative |
| CYP2C19 | Negative |
| FLG | Negative |
| DBNDD1 | Negative |
| DSG3 | Negative |
| AMOT | Negative |
| RIMKLB | Negative |
| SERPINB8 | Negative |
| SLC6A20 | Negative |
| CNN3 | Negative |
| SEMA5A | Negative |
| DUXAP8 | Negative |
| SMIM24 | Negative |
| ADH1C | Negative |
| MGP | Negative |
| MATN2 | Positive |
| CD81 | Positive |
| ANTXR2 | Positive |
| RUNX3 | Positive |
| KYNU | Positive |
| DHRS9 | Positive |
| RAC2 | Positive |
| COL4A2 | Positive |
| IGFBP1 | Positive |
| SYCE1 | Positive |
| BMP7 | Positive |
| TGFBI | Positive |
| TFAP2C | Positive |
| PRDM8 | Positive |
| HOTAIRM1 | Positive |
| MYBL1 | Positive |
| CDHR5 | Positive |
| COL17A1 | Positive |
| TMTC1 | Positive |
| LINC01169 | Positive |
| BEX3 | Positive |
| TRPV2 | Positive |
| LXN | Positive |
| MYADM | Positive |
| NCCRP1 | Positive |
| MLPH | Positive |
| SLC16A3 | Positive |
| C1QTNF1 | Positive |
| FAXDC2 | Positive |
| FOXQ1 | Positive |
| FLNA | Positive |
| IGFBP7 | Positive |
| SERPINE1 | Positive |
| CHST11 | Positive |
| FKBP5 | Positive |
| VIM | Positive |
| LDOC1 | Positive |
| LDHB | Positive |
| XKR9 | Positive |
| KLK11 | Positive |
| SCNN1G | Positive |
| HAL | Positive |
| LINC00960 | Positive |
| PRAME | Positive |
| CLMP | Positive |
| AXL | Positive |
| RGS20 | Positive |
| EDIL3 | Positive |
| MAPRE3 | Positive |
| CDA | Positive |
| MPP1 | Positive |
| ZNF418 | Positive |
| TGM2 | Positive |
| KLK7 | Positive |
| GCNT3 | Positive |
| PAX9 | Positive |
| ALOX15B | Positive |
| TOX2 | Positive |
| NPBWR1 | Positive |
| PLCB1 | Positive |
| DCBLD1 | Positive |
| TMX3 | Positive |
| PRR5L | Positive |
| CD40 | Positive |
| FIGN | Positive |
| MAFB | Positive |
| COL4A5 | Positive |
| COPZ2 | Positive |
| RUNX2 | Positive |
| ACKR2 | Positive |
| TRPS1 | Positive |
| NNMT | Positive |
| TIMP3 | Positive |
| FSTL1 | Positive |
| HOXA13 | Positive |
| EPGN | Positive |
| LBH | Positive |
| LY6K | Positive |
| RFX8 | Positive |
| HOXB8 | Positive |
| DKK1 | Positive |
| PLCB2 | Positive |
| APBB1 | Positive |
| PCDH7 | Positive |
| NTSR1 | Positive |
| BMF | Positive |
| ZNF365 | Positive |
| NID1 | Positive |
| LINC00654 | Positive |
| CES4A | Positive |
| BNIP3 | Positive |
| PRSS21 | Positive |
| CYP2B7P | Positive |
| TNC | Positive |
| APOD | Positive |
| KLK6 | Positive |
| OSBP2 | Positive |
| ECHDC3 | Positive |
| C2 | Positive |
| FAM83A | Positive |
| MIA | Positive |
| FPR1 | Positive |
| LGALS1 | Positive |
| STMN3 | Positive |
| RAB34 | Positive |
| GJB2 | Positive |
| ADAM19 | Positive |
| FLRT2 | Positive |
| IRX3 | Positive |
| COL18A1 | Positive |
| HOXB13 | Positive |
| KLHL4 | Positive |
| TRPM2 | Positive |
| ARMCX1 | Positive |
| CADM1 | Positive |
| CHGB | Positive |
| PREX1 | Positive |
| KRT6A | Positive |
| GABRE | Positive |
| POPDC3 | Positive |
| HOXC12 | Positive |
| GLI3 | Positive |
| C11orf86 | Positive |
| SNAI2 | Positive |
| WWC3 | Positive |
| CASC9 | Positive |
| VGLL3 | Positive |
| ZNF415 | Positive |
| MIR100HG | Positive |
| JCAD | Positive |
| SLCO2B1 | Positive |
| RBP4 | Positive |
| HOXC10 | Positive |
| NRCAM | Positive |
| EFCAB1 | Positive |
| LGALS4 | Positive |
| CLDN10 | Positive |
| ARL11 | Positive |
| CDYL2 | Positive |
| TNS4 | Positive |
| DACT2 | Positive |
| CLEC4O | Positive |
| COL5A2 | Positive |
| HOXC13 | Positive |
| CPE | Positive |
| HAS2 | Positive |
| HIF3A | Positive |
| ZNF114 | Positive |
| PMEPA1 | Positive |
| CCN6 | Positive |
| LYPD3 | Positive |
| PTPRS | Positive |
| COL6A1 | Positive |
| HORMAD1 | Positive |
| MDFIC | Positive |
| ANGPTL2 | Positive |
| KRT6B | Positive |
| BTBD6 | Positive |
| LNCAROD | Positive |
| EPHA4 | Positive |
| SLC44A5 | Positive |
| IGFL2 | Positive |
| TRHDE | Positive |
| MUC3A | Positive |
| CDKN2B | Positive |
| TMEM45A | Positive |
| WDR72 | Positive |
| CCBE1 | Positive |
| L1CAM | Positive |
| BASP1 | Positive |
| IGF2 | Positive |
| GASK1B | Positive |
| COL5A1 | Positive |
| EFEMP1 | Positive |
| LINC00668 | Positive |
| SERPINE2 | Positive |
| LINC01819 | Positive |
| QPCT | Positive |
| ROS1 | Positive |
| COL6A2 | Positive |
| IGF2BP1 | Positive |
| FN1 | Positive |

In this table, when the correlation column indicates "Positive", thus presence of the corresponding RNA transcript is significantly correlated to sensitivity of cells to Oxaliplatin. On the opposite, when the correlation column indicates "Negative", thus absence of the corresponding RNA transcript is significantly correlated to sensitivity of cells to Oxaliplatin.

Fluoro-uracil (5-FU) (ATCC: L01BC02, 5-fluoro-2,4(1H,3H)-pyrimidinedion) is used to treat a number of types of cancer including pancreatic cancer. It is in the antimetabolite family of drugs and more particularly a pyrimidine analog.

The inventors determined that the sensitivity RNA signature of 5-FU is as described in Table 3.

**Table 3: Sensitivity RNA signature of 5-FU**

| **Gene** | **Correlation** |
|---|---|
| TSHZ2 | Negative |
| MGP | Negative |
| NR5A2 | Negative |
| EPHB6 | Negative |
| VCAN | Negative |
| FGF19 | Negative |
| IL7R | Negative |
| SERPINA3 | Negative |
| AKR1B10 | Negative |
| CA9 | Negative |
| ITGA9 | Negative |
| SUCNR1 | Negative |
| CST4 | Negative |
| WNT7A | Negative |
| AQP5 | Negative |
| WFDC2 | Negative |
| SLCO4A1 | Negative |
| ALB | Negative |
| EDIL3 | Positive |
| CDON | Positive |
| CCDC152 | Positive |
| TNNT1 | Positive |
| PPP1R1B | Positive |
| AQP1 | Positive |
| ITLN1 | Positive |
| SELENOP | Positive |
| SLC13A5 | Positive |
| ADIRF | Positive |
| TNFRSF6B | Positive |
| SRGN | Positive |
| CSPG4 | Positive |
| BCAT1 | Positive |
| DMBT1 | Positive |
| PADI2 | Positive |
| FER1L6 | Positive |
| ANPEP | Positive |
| TIMP3 | Positive |
| QPRT | Positive |
| MUC6 | Positive |

In this table, when the correlation column indicates "positive", thus presence of the corresponding RNA transcript is significantly correlated to sensitivity of cells to 5-FU. On the opposite, when the correlation column indicates "Negative", thus absence of the corresponding RNA transcript is significantly correlated to sensitivity of cells to 5-FU.

Irinotecan (ATCC: L01XX19) is an anticancerous drug in the topoisomerase inhibitor family. It is a prodrug from which the active metabolite inhibits topoisomerase 1, which results in DNA damage and cell death.

The inventors determined that the sensitivity RNA signature of Irinotecan is as described in Table 4.

**Table 4: Sensitivity RNA signature of Irinotecan**

| **Gene** | **Correlation** |
|---|---|
| TNC | Negative |
| MYBPC1 | Negative |
| AKR1C2 | Negative |
| IFI44L | Negative |
| ANPEP | Negative |
| CLDN10 | Positive |
| SLC28A3 | Positive |
| CFTR | Positive |
| LY6D | Positive |
| TFF3 | Positive |
| MTCL1 | Positive |
| TNS1 | Positive |
| SEMA3E | Positive |
| VTCN1 | Positive |
| PROM1 | Positive |
| MME | Positive |
| PRODH | Positive |
| DUOXA2 | Positive |
| GPC4 | Positive |
| KRT17 | Positive |
| VGLL3 | Positive |
| CXCL6 | Positive |
| PDE3A | Positive |
| KLK7 | Positive |
| SLC26A9 | Positive |

In this table, when the correlation column indicates "Positive", thus presence of the corresponding RNA transcript is significantly correlated to sensitivity of cells to Irinotecan. On the opposite, when the correlation column indicates "Negative", thus absence of the corresponding RNA transcript is significantly correlated to sensitivity of cells to Irinotecan.

Taxanes are a class of diterpenes. They were originally identified from plants of the genus *Taxus,* and feature a taxadiene core. Numerous taxanes are used as anticancerous treatment such as Paclitaxel (Taxol), docetaxel (Taxotere) or Cabazitaxel. The principal mechanism of action of the taxane class of drugs is the disruption of microtubule function.

Preferably, said taxane according to the invention is docetaxel, paclitaxel or nab-paclitaxel.

The inventors determined that the sensitivity RNA signature of taxane is as described in Table 5.

**Table 5: Sensitivity RNA signature of taxane**

| **Gene** | **Correlation** |
|---|---|
| MMP1 | Positive |
| SLC26A9 | Positive |
| AZGP1 | Positive |
| VCAN | Positive |
| COL6A2 | Positive |
| CLDN18 | Positive |
| CEACAM7 | Positive |
| VNN2 | Positive |
| PRAME | Positive |
| TFF2 | Positive |
| SPRR1B | Positive |
| MME | Positive |
| SYNE1 | Positive |
| CDH6 | Positive |
| LINC01088 | Positive |
| ATP13A4 | Positive |
| SATB1 | Positive |
| GPC3 | Positive |
| SEMA6D | Positive |
| LCP1 | Positive |
| G0S2 | Positive |
| CLU | Positive |
| IL33 | Positive |
| PRSS23 | Positive |
| SERPINA6 | Positive |
| THBS1 | Positive |
| DIO3OS | Positive |
| TPPP3 | Positive |
| MEX3C | Positive |
| TFF3 | Positive |
| KRT81 | Positive |
| SMAD4 | Positive |
| ZNF702P | Positive |
| UGT1A10 | Positive |
| SPOCK2 | Positive |
| HBEGF | Positive |
| IL6R | Positive |
| LAMC2 | Positive |
| FBXO2 | Positive |
| MGP | Positive |
| SLCO2A1 | Positive |
| ANPEP | Positive |
| EML1 | Positive |
| AQP1 | Positive |
| UST | Positive |
| MAPK8IP2 | Positive |
| CEACAM18 | Positive |
| ZSCAN18 | Positive |
| FAM171A1 | Positive |
| MYADM | Positive |
| DPP4 | Positive |
| CASP1 | Positive |
| GSTM1 | Positive |
| COL5A2 | Positive |
| VWDE | Positive |
| PAX7 | Positive |
| REG4 | Positive |
| CNTFR | Positive |
| CA9 | Positive |
| SDK1 | Positive |
| LIMCH1 | Positive |
| PCLO | Positive |
| ADGRF1 | Positive |
| TNFRSF6B | Positive |
| RGS2 | Positive |
| LAMB1 | Positive |
| SLC19A3 | Positive |
| FLNA | Positive |
| GABRB3 | Positive |
| DSEL | Positive |
| TM4SF4 | Positive |
| TSPAN18 | Positive |
| CCN3 | Positive |
| SULT1C2 | Positive |
| SCG5 | Positive |
| DPY19L2 | Positive |
| SPRR3 | Positive |
| LDLRAD4 | Positive |
| C6orf141 | Positive |
| ADH1C | Positive |
| GABRP | Positive |
| PLXDC2 | Positive |
| DQX1 | Positive |
| TNFRSF1B | Positive |
| KCNH3 | Positive |
| STMN3 | Positive |
| CES1 | Positive |
| SCN9A | Positive |
| UBD | Positive |
| MEIS2 | Positive |
| KISS1 | Positive |
| ZNF43 | Positive |
| MDK | Positive |
| RPE65 | Positive |
| NMNAT2 | Positive |
| CXCL17 | Positive |
| CYFIP2 | Positive |
| SIDT1 | Positive |
| ANKRD40CL | Positive |
| RIMKLB | Positive |
| ZNF542P | Positive |
| CXCL1 | Positive |
| COL22A1 | Positive |
| RERG | Positive |
| KCNQ3 | Positive |
| DNAJB13 | Positive |
| FBXO17 | Positive |
| BTNL9 | Positive |
| XRCC4 | Positive |
| SERPINB7 | Positive |
| SEMA6A | Positive |
| GJC1 | Positive |
| TM4SF5 | Positive |
| ZNF568 | Positive |
| TUBB3 | Positive |
| GRHL3 | Positive |
| B3GAT1 | Positive |
| TMPRSS3 | Positive |
| SUCNR1 | Positive |
| TMEFF2 | Positive |
| ZNF486 | Positive |
| LINC01234 | Positive |
| PRDM16 | Positive |
| MPP1 | Positive |
| GGT6 | Positive |
| PLSCR4 | Positive |
| LINC01819 | Positive |
| CRMP1 | Positive |
| FUT3 | Positive |
| PROM2 | Positive |
| CFTR | Positive |
| GRM5 | Positive |
| NR0B2 | Positive |
| CA2 | Positive |
| FABP3 | Positive |
| MPDZ | Positive |
| BEX4 | Positive |
| KRT10 | Positive |
| NRP2 | Positive |
| TUSC3 | Positive |
| MXRA5 | Positive |
| WDR72 | Positive |
| CYP2B6 | Positive |
| SECTM1 | Positive |
| MCAM | Positive |
| FERMT2 | Positive |
| KRT5 | Positive |
| ELOVL2 | Negative |
| CDYL2 | Negative |
| PGGHG | Negative |
| ZNF345 | Negative |
| PRPH | Negative |
| SERPINA5 | Negative |
| CACNA1C | Negative |
| KLK8 | Negative |
| LGALS2 | Negative |
| ABCG8 | Negative |
| LTBP1 | Negative |
| KLK9 | Negative |
| GRID1 | Negative |
| SYCP2 | Negative |
| ZNF154 | Negative |
| MAL2 | Negative |
| ABCA6 | Negative |
| TTTY14 | Negative |
| TSPAN8 | Negative |
| NXN | Negative |
| NECAB1 | Negative |
| VIM | Negative |
| COL8A1 | Negative |
| ZNF257 | Negative |
| CTNND2 | Negative |
| PRSS3 | Negative |
| PRKD1 | Negative |
| ZIK1 | Negative |
| B4GALNT4 | Negative |
| AKAP12 | Negative |
| LINC02532 | Negative |
| RNF144A | Negative |
| KLHDC7A | Negative |
| KLK10 | Negative |
| EREG | Negative |
| RAB3B | Negative |
| UTRN | Negative |
| THSD7A | Negative |
| DUSP27 | Negative |
| NR5A2 | Negative |
| ITM2A | Negative |
| ZFP28 | Negative |
| ABCG5 | Negative |
| PFN2 | Negative |
| MAOB | Negative |
| AGMO | Negative |
| SERPINB2 | Negative |
| TDRP | Negative |
| C8orf88 | Negative |
| ACHE | Negative |
| MPP6 | Negative |
| OLFM2 | Negative |
| ENAM | Negative |
| ZNF469 | Negative |
| CAPN9 | Negative |
| TENM2 | Negative |
| GJA1 | Negative |
| CXCL8 | Negative |
| ZNF470 | Negative |
| LNCOC1 | Negative |
| FGFBP1 | Negative |
| TGM3 | Negative |
| MT2A | Negative |
| LAMA4 | Negative |
| ZNF518B | Negative |
| LNCAROD | Negative |
| USP32P1 | Negative |
| IGF2BP1 | Negative |
| ADAMTS1 | Negative |
| GCNT2 | Negative |
| SYT11 | Negative |
| MUC3A | Negative |
| PTGER2 | Negative |
| CCDC144A | Negative |
| SNAP25 | Negative |
| LY6K | Negative |
| PIK3C2G | Negative |
| VAV1 | Negative |
| SRGN | Negative |
| COL18A1 | Negative |
| EIF1AY | Negative |
| APBA2 | Negative |
| FRMD3 | Negative |
| CADM1 | Negative |
| FAT4 | Negative |
| UTY | Negative |
| PRDM5 | Negative |
| TNFAIP6 | Negative |
| RNF152 | Negative |
| CDC25B | Negative |
| HPGD | Negative |
| FABP6 | Negative |
| TRIM29 | Negative |
| ACE2 | Negative |
| ECM1 | Negative |
| TTN | Negative |
| CMTM3 | Negative |
| ZFP82 | Negative |
| ALDH1A2 | Negative |
| GATA3 | Negative |
| CLDN2 | Negative |
| LINGO1 | Negative |
| DKK1 | Negative |
| MCOLN3 | Negative |
| KCTD15 | Negative |
| CHST9 | Negative |
| PADI1 | Negative |
| MTAP | Negative |
| KDM5D | Negative |
| MICB | Negative |
| CYS1 | Negative |
| TM4SF20 | Negative |
| ROR1 | Negative |
| KCNJ15 | Negative |
| NPY1R | Negative |
| LRRC19 | Negative |
| PRKY | Negative |
| ZFY | Negative |
| GYG2P1 | Negative |
| USP9Y | Negative |
| KLK6 | Negative |
| SLC7A9 | Negative |
| MRC2 | Negative |
| TRPM2 | Negative |
| TXLNGY | Negative |
| PLCB1 | Negative |
| NHS | Negative |
| IL6 | Negative |
| UCP2 | Negative |
| A2ML1 | Negative |
| KRT6B | Negative |
| DDX3Y | Negative |
| PDGFD | Negative |
| CD163L1 | Negative |
| DHRS9 | Negative |
| BST2 | Negative |
| ALPK3 | Negative |
| PLA2G3 | Negative |
| WIF1 | Negative |
| ZFP30 | Negative |
| MAP1B | Negative |
| PLCB2 | Negative |
| KLK7 | Negative |
| PDE4B | Negative |
| PLEKHS1 | Negative |
| SLC28A3 | Negative |
| SCTR | Negative |
| NIBAN1 | Negative |
| MGAM2 | Negative |
| PTGS2 | Negative |
| CAPN12 | Negative |
| BICC1 | Negative |
| PRR29 | Negative |
| ARHGAP6 | Negative |
| TGFB2 | Negative |
| OLFM4 | Negative |
| SCEL | Negative |
| RPS4Y1 | Negative |
| GLI2 | Negative |
| C5 | Negative |
| ICAM2 | Negative |
| CADPS | Negative |
| CARMIL2 | Negative |
| PGBD5 | Negative |
| TRPV4 | Negative |
| NTS | Negative |
| ZBED2 | Negative |
| MUCL3 | Negative |
| GPRC5B | Negative |
| CFI | Negative |
| SULT1E1 | Negative |
| TRIM22 | Negative |
| HPN | Negative |

In this table, when the correlation column indicates "Positive", the presence of the corresponding RNA transcript is significantly correlated to sensitivity of cells to taxanes, and more preferably to docetaxel, paclitaxel and/or nab-paclitaxel. On the opposite, when the correlation column indicates "Negative", thus absence of the corresponding RNA transcript is significantly correlated to sensitivity of cells to taxanes, and more preferably to docetaxel, paclitaxel and/or nab-paclitaxel.

In an embodiment of the invention, a RNA signature of PDAC cells corresponds to any one of tables 1 to 5, when all RNA transcript of the sensitivity signature whose presence is positively correlated to sensitivity to the treatment in the table are detected in PDAC cells, and all RNA transcript whose absence is positively correlated to sensitivity to the treatment in the table is not detected in PDAC cells.

In another embodiment, correspondence between RNA signature of PDAC cells and any one of tables 1 to 5 can be evaluated by a score. The algorithm calculating the score can be statistically determined by cohort studies. The more the signature of PDAC cells is close to the sensitivity RNA signature of a treatment, the higher is the score.

When the score is higher than a predetermined value (hereby threshold), the patient is determined as being sensitive to the treatment. Said threshold can be defined by various methods such as a mean value observed in a population or cohort, or by ROC analysis. According to the invention, the threshold may be determined by a plurality of samples, preferably more than 5, 50, 100, 200 or 500 samples.

Such score can also be used for stratification of patient population. For example, in one embodiment of the invention, the quartile of the population with the lowest score is considered not sensitive to the treatment, the quartile of the population with the highest score is considered highly sensitive to the treatment, and the rest of the population is considered as normally sensitive.

The polychemotherapy regimen FOLFIRINOX is the combination of three different chemotherapy drugs and a vitamin: Fluoro-uracil (5-FU), Irinotecan, and Oxaliplatin. It also includes folinic acid (Leucovorin). It is one of the main chemotherapy treatments for pancreatic ductal adenocarcinoma.

In a particular embodiment, if a patient is determined sensitive to oxaliplatin, 5-FU and/or irinotecan by the methods of the invention, said patient can be considered sensitive to the FOLFIRINOX treatment.

In another embodiment, a score can be calculated in function of the correspondence between the RNA signature of PDAC cells of the patient and Tables 2, 3 and/or 4 to determine if a patient is sensitive to the FOLFIRINOX treatment.

In a preferred embodiment of the method according to the invention, RNA transcripts of the sensitivity RNA signature of a treatment are RNA transcript of genes of table 1, and/or of table 2, and/or of table 3, and/or of table 4 and/or of table 5.

### Medical indications

On another aspect the invention concerns Gemcitabine, Oxaliplatin, 5 fluoro-uracil (5-FU), Irinotecan, a taxane or a combination thereof, for use for treating PDAC in a patient, wherein said patient has been determined as having a PDAC sensitive to gemcitabine, oxaliplatin, 5 fluoro-uracil (5-FU), irinotecan, a taxane or a combination thereof.

The invention also concerns a method of treatment of PDAC in a patient comprising the administration of Gemcitabine, Oxaliplatin, 5 fluoro-uracil (5-FU), Irinotecan, a taxane or a combination thereof, wherein said patient having a PDAC is sensitive to gemcitabine, oxaliplatin, 5 fluoro-uracil (5-FU), irinotecan, a taxane or a combination thereof.

In an embodiment a therapeutically effective amount of Gemcitabine, Oxaliplatin, 5 fluoro-uracil (5-FU), Irinotecan, a taxane or a combination thereof is administered to a patient in need thereof.

As used herein, the expression "therapeutically effective amount" means a sufficient amount of a compound to treat a specific disease, at a reasonable benefit/risk ratio applicable to any medical treatment. It will be understood, however, that the total daily usage of the compounds of the present invention will be decided by attending physician within the scope of sound medical judgment. The specific therapeutically effective dose level for any particular subject will depend upon a variety of factors including the disorder being treating and the severity of the disorder, activity of the specific compounds employed, the specific combinations employed, the age, body weight, general health, sex and diet of the subject, the time of administration, route of administration, the duration of the treatment, drugs used in combination or coincidental with the specific compounds employed, and like factors well known in the medical arts.

Preferably, the patient having a PDAC has been determined sensitive to gemcitabine, oxaliplatin, 5 fluoro-uracil (5-FU), irinotecan, a taxane or a combination thereof according to one method of the invention.

### Method for determining a suitable treatment

The present invention provides a method, preferably an *ex vivo* method, for determining a suitable treatment for a patient having pancreatic ductal adenocarcinoma (PDAC), comprising:
a) determining if the patient is sensitive to a treatment using the method of disclosed herein, and
b) deducing a suitable treatment for the patient.

By "suitable treatment", it is meant a treatment that the patient has higher chance of responding to, preferably the patient having PDAC is sensitive to said treatment as the. A suitable treatment has the potential to improve survival by matching drugs to likely responders, but also to reduce adverse effects by avoiding unnecessary highly toxic regimens.

Preferably, in step b) the suitable treatment for the patient is a treatment from which the patient is determined as sensitive or highly sensitive. Said suitable treatment can be the combination of several therapeutic drugs, such as the FOLFIRINOX treatment.

In an embodiment of the invention, step a) comprises determining if the patient is sensitive to a treatment with gemcitabine, oxaliplatin, 5 fluoro-uracil (5-FU), irinotecan, a taxane or a combination, preferably using the method of the invention.

### Kit

The invention also relates to a kit for determining if a patient having pancreatic ductal adenocarcinoma (PDAC) is sensitive to a treatment with gemcitabine, oxaliplatin, 5 fluoro-uracil (5-FU), irinotecan, a taxane or a combination thereof, comprising means for detecting RNA of genes of tables 1, 2, 3, 4 or 5.

According to the invention "means for detecting RNA of genes of tables 1, 2, 3, 4 or 5, comprises chip, microchip, RNA-sequencing, probes and/or a set of primers, preferably it comprises chip, microchip, probes and/or a set of primers. In a particular embodiment, said chip or microchip comprises probes. Probes and primers of the kit, of the chip or of the microchip of the kit are preferably specific to RNA of genes of tables 1, 2, 3, 4 or 5.

In an embodiment, said kit further comprises means for taking a sample of PDAC cells, such as means for biopsy.

The invention also concerns the use of a kit according to the invention for determining if a patient having pancreatic ductal adenocarcinoma (PDAC) is sensitive to a treatment with gemcitabine, oxaliplatin, 5 fluoro-uracil (5-FU), irinotecan, a taxane or a combination thereof.

The invention also concerns the use of a kit according to the invention for determining a suitable treatment for a patient having PDAC.

The present invention will be further illustrated by the figures and examples below.

### Brief description of the figures

Figure 1: A. PDPCC training cohort correlation among ICA2 component and Gemcitabine AUC. B. PDPCC validation cohort correlation after ICA2 projection.
Figure 2: A. PDX validation cohort correlation after ICA2 projection. B. BDPO validation cohort correlation after ICA2 projection. C. CACL validation cohort correlation after ICA2 projection.
Figure 3: A. PDPCC training cohort correlation among ICA18 component and Oxaliplatin AUC. B. PDPCC validation cohort correlation after ICA18 projection.
Figure 4: A. PDX validation cohort correlation after ICA18 projection. B. BDPO validation cohort correlation after ICA18 projection. C. CACL validation cohort correlation after ICA18 projection.
Figure 5: A. PDPCC training cohort correlation among ICA12 component and 5-FU AUC. B. PDPCC validation cohort correlation after ICA12 projection.
Figure 6: A. PDX validation cohort correlation after ICA12 projection. B. BDPO validation cohort correlation after ICA12 projection. C. CACL validation cohort correlation after ICA12 projection.
Figure 7: A. PDPCC training cohort correlation among ICA1 component and Irinotecan AUC. B. PDPCC validation cohort correlation after ICA1 projection.
Figure 8: A. PDX validation cohort correlation after ICA1 projection. B. BDPO validation cohort correlation after ICA1 projection. C. CACL validation cohort correlation after ICA1 projection.
Figure 9: A. PDPCC training cohort correlation among ICA12 component and Docetaxel AUC. B. PDPCC validation cohort correlation after ICA12 projection.
Figure 10: A. PDX validation cohort correlation after ICA12 projection. B. BDPO validation cohort correlation after ICA12 projection. C. CACL validation cohort correlation after ICA12 projection.

### Examples:

### Example 1:

The purpose of this study was to determine if an efficient set of RNAs can be determined to predict the Gemcitabine, 5-FU, Oxaliplatin, Irinotecan and taxanes sensitivity in PDAC, and/or to be used for selecting sensitive patients to predict their more efficient treatment.

### Methods

### Patient's cohort

Patients with PDAC diagnosis between February 2011 and September 2015 were included in this study under the clinical trial "Predictive Biomarkers of Therapeutic Response in Pancreatic Tumors".

### Patient derived tumor xenograft (PDX) generation

Animal experiments were conducted following institutional guidelines and "Plateforme de Stabulation et d'Experimentation Animale" (PSEA, Scientific Park of Luminy, Marseille) approval. PDAC tissues were fragmented, mixed with 100 µL of Matrigel and implanted with a 10 gauge trocar (Innovative Research of America, Sarasota, FL) in the subcutaneous right upper flank of an anesthetized male NMRI-nude mouse (Swiss Nude Mouse Crl: NU(Ico)-Foxn1nu; Charles River Laboratories, Wilmington, MA). Once xenografts reached 1 cm³, they were removed and passed to NMRI-nude mice.

### PDX-derived primary cell culture (PDPCC) isolation

Xenografts obtained from mice were split into several small pieces and used for cell culture. These fragments were processed in a biosafety chamber: after fine mincing, they were treated with collagenase type V (ref C9263; Sigma-Aldrich, St. Louis, MO) and trypsin/EDTA (ref 25200-056; Gibco, Life Technologies, Grand Island, NY) and were suspended in Dulbecco's modified Eagle's medium supplemented with 1% w/w penicillin/streptomycin (Gibco, Life Technologies) and 10% fetal bovine serum (Lonza Inc., Walkersville, MD). After centrifugation, cells were resuspended in serum-free ductal media at 37°C in a 5% CO2 incubator. Amplified cells were stored in liquid nitrogen. Cells were weaned from antibiotics for ≥48 hours before testing.

### Biopsy-derived pancreatic organoids (BDPO) generation

Tumoral cells were obtained from the PDAC biopsies through slight digestion with the Tumor Dissociation Kit (Miltenyi Biotec) at 37°C for 5 minutes. Isolated cells were placed into 12-well plates coated with 150 µL growth factor-reduced Matrigel (Corning) and cultured with advanced DMEM/F12 supplemented with HEPES (10 mmol/L; Thermo Fisher Scientific), human recombinant FGF10 (100 ng/m; PeproTech), human recombinant EGF (50 ng/mL; PeproTech), human recombinant Noggin (100 ng/mL; Bio-Techne), human Gastrin 1 (10 nmol/L; Sigma-Aldrich, Inc.), Nicotinamide (10 mmol/L; Sigma-Aldrich, Inc.), N-acetylcysteine (1.25 mmol/L; Sigma-Aldrich, Inc.), B27 (Thermo Fisher Scientific), A83-01 (500 nmol/L; Bio-Techne), and Y27632 (10.5 µmol/L; Bio-Techne). The plates were incubated at 37°C in a 5% CO2 incubator, and the media changed every 3 to 4 days.

### Chemograms in PDPCC

PDPCC were screened for chemosensitivity with five cytotoxic drugs: Gemcitabine (Eli Lilly & Co., Indianapolis, IN), Oxaliplatin (Hospira, Lake Forest, IL) and active Irinotecan metabolite named 7-ethyl 10-hydroxycamptothecin or SN-38 (11406; Sigma-Aldrich), 5-FU (Teva Pharmaceutical Industries, Petah Tikva, Israel) and Docetaxel (Sanofi, Bridgewater, NJ). These cells were treated for 72 hours with increasing concentrations of chemotherapeutic drugs ranging from 0 to 1000 µmol/L. Five thousand cells were plated per well in 96-well plates in serum free defined media. Twenty-four hours later, the media was supplemented with increasing concentrations of drugs and incubated for 72 hours. Each experiment was performed in triplicate and repeated at least three times. Cell viability was estimated after the addition of the PrestoBlue cell viability reagent (Life Technologies) for 3 hours following the supplier's protocol. Cell viability was measured on days 0 and 3 to calculate the replication rate of the cells. Area under the curve (AUC) was calculated.

### in vivo PDX models of chemosensitivity in PDX

Forty PDAC were included in this study to derive PDX that were tested for drug chemosensitivity. Each PDX corresponding to one patient was inoculated into 8 nude NMRI mice for each treatment. A total of 672 (8 x 6 x 14) animals were utilized. When the PDX for each patient reached 200 mm3 animals were randomized and treated with Gemcitabine (120 mg/kg every third day for four administrations in the tail vein, n = 8), Irinotecan (22 mg/kg every second day for three administrations in the tail vein, n = 8), Docetaxel (5 mg/kg every second day for three administrations in the tail vein, n = 8) 5-FU (56 mg/kg every four days with two administrations in the tail vein, n = 8) and Oxaliplatin (5 mg/kg every fourth day for two administrations in the tail vein, n = 8) or vehicle (NaCl 0.9% n = 8). Chemotherapy sensitivity was revealed by tumor growth monitoring. Tumor size was measured with a Vernier caliper twice weekly, and the tumor volume was calculated with the equation v = (length/width2)/2. The score of tumor growth was calculating for each PDX and for each treatment as the AUC of treated animals over the AUC of untreated animals.

### BDPO chemosensitivity

BDPOs were disaggregated with accutase (Thermo Fisher Scientific), and 1,000 cells/well were plated in a 96-well round bottom ultra-low plate (Corning) with the medium described above. Twenty-four hours later, the media was supplemented with increasing concentrations of Gemcitabine, Oxaliplatin, SN-38, 5-FU and Docetaxel and incubated for 72 hours. Cell viability was measured with CellTiter-Glo 3D (Promega) reagent quantified using the plate reader Tristar LB941 (Berthold Technologies). Each experiment was repeated at least three times. Eight increasing concentrations of cytotoxic drugs were used ranging from 0 to 1 mmol/L. Values were normalized and expressed as the percentage of the control (vehicle), which represent 100% of normalized fluorescence.

### Commercially available cell lines (CACL) microarray and chemo-sensitivity data analysis

CEL microarray files for 21 pancreatic cancer CACL were downloaded from 209 ArrayExpress database (E-MTAB-3610). Microarray data was processed following the workflow detailed in the maEndToEnd R package. Briefly, oligo R package was used to read, background subtraction and normalization of probe set intensity applied Robust Multi-array Analysis (RMA). Then, common cell lines with the Dependency Map chemosensitivity database (DepMap) were used for chemo-sensitivity signature validation. For each cell line, AUC was extracted from PRISM Repurposing Secondary Screen 19Q4 database.

### RNA isolation method and expression analysis

PDPCC total RNA was extracted using RNeasy Mini Kit (Qiagen). RNA libraries were prepared (Illumina TruSeq RNA v2) and run on the Illumina High Seq-2000 for 101 bp paired end reads. Gene expression profiles were obtained using the MAP-RSeq v.1.2.1 workflow. MAP-RSeq consists of alignment with TopHat 2.0.626 against the human hg19 genome build and gene counts with the HTSeq software 0.5.3p9 using gene annotation files obtained from Illumina. Gene counts were normalized using upper quartile.

### Chemo-sensitivity signature generation

Initially, we applied robust PCA to decompose the PDX and PDPCC expression matrix in low rank and sparse, representing the basal and perturbation gene sets, respectively. In parallel, each drug's training cohort was determined by selecting the samples between AUC's percentiles (P). The sensitive group was allocated in the P10 and P25, whereas the range among the P75 and P90 defined the resistant group. The chemo-sensitivity components were calculated using independent component analysis (ICA) on the robust PCA sparse matrix, previous integration of PDX, and PDPCC gene expression of each training cohort. Spearman correlation was performed to determine the most relevant component associated with the chemo-sensitivity profiles. All statistical analysis was performed using the R statistical suite.

### Results

### Gemcitabine chemo-sensitivity signature

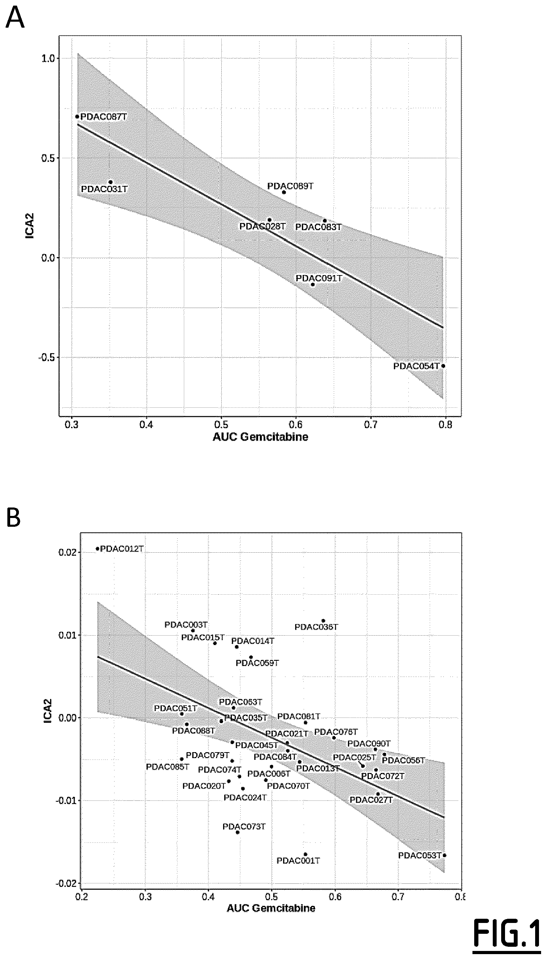

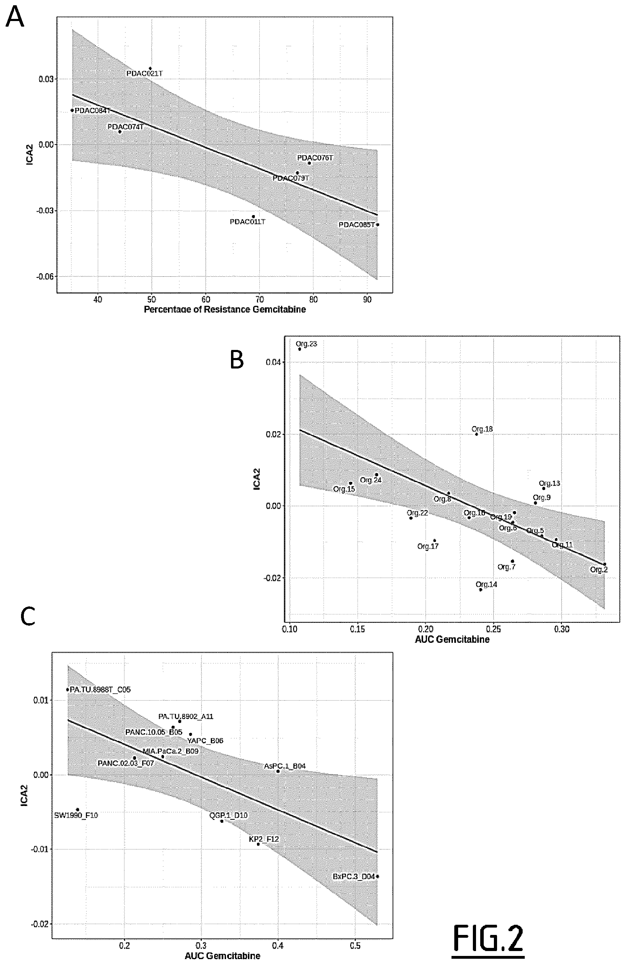

The reference component (ICA2) was extracted from PDPCC using AUC as chemo-sensitivity parameter (Figure 1A; r = -0.93; P = 0.002; Table 1). The selected component was validated in an independent PDPCC cohort (Figure 1B; r = -0.52; P = 0.003). Furthermore, PDX (Figure 2A; r = -0.79; P = 0.03), BDPO (Figure 2B; r = -0.64; P = 0.005) and CACL (Figure 2C; r = -0.66; P = 0.03) chemo-sensitivity was predicted applying ICA2 on the expression matrices.

### Oxaliplatin chemo-sensitivity signature

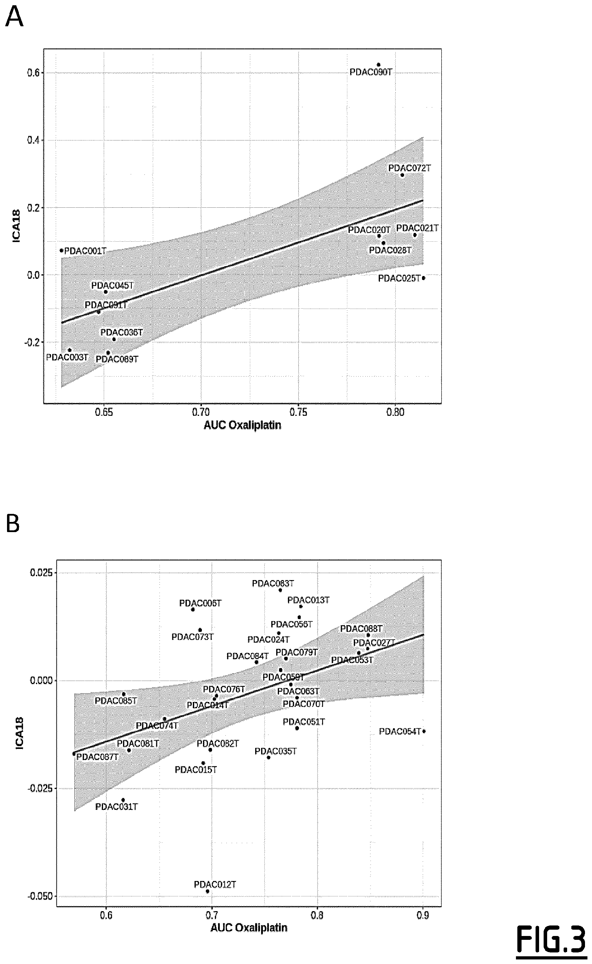

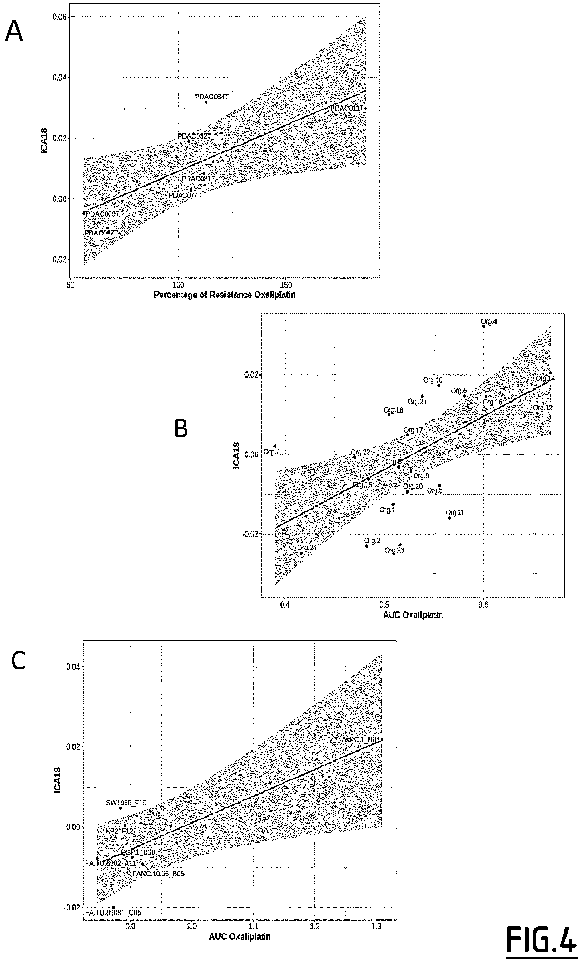

Oxaliplatin selected component (ICA18) correlates positively with the PDPCC training cohort AUC (Figure 3A; r = 066; P = 0.02; Table 2). ICA18 was applied in an independent PDPCC cohort (Figure 3B; r = 0.44; P = 0.02) and PDX (Figure 4A; r = 0.82; P = 0.02). To further confirm the signature robustness, ICA18 component was projected in both, BDPO and CACL, displaying a significant correlation with the AUC, with a coefficient of 0.59 (Figure 4B; P = 0.03) and 0.81 (Figure 4C; P = 0.005), respectively.

### 5-FU chemo-sensitivity signature

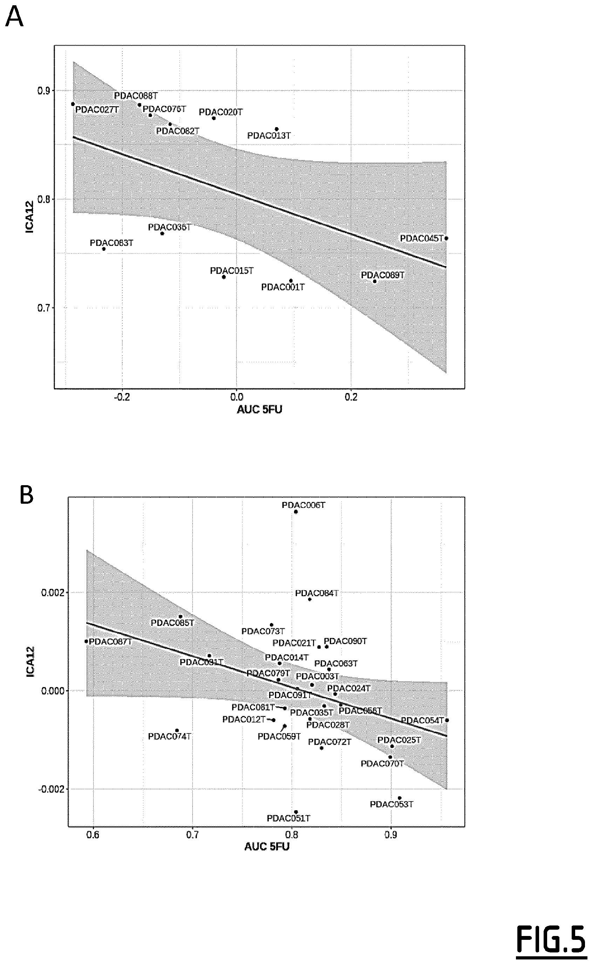

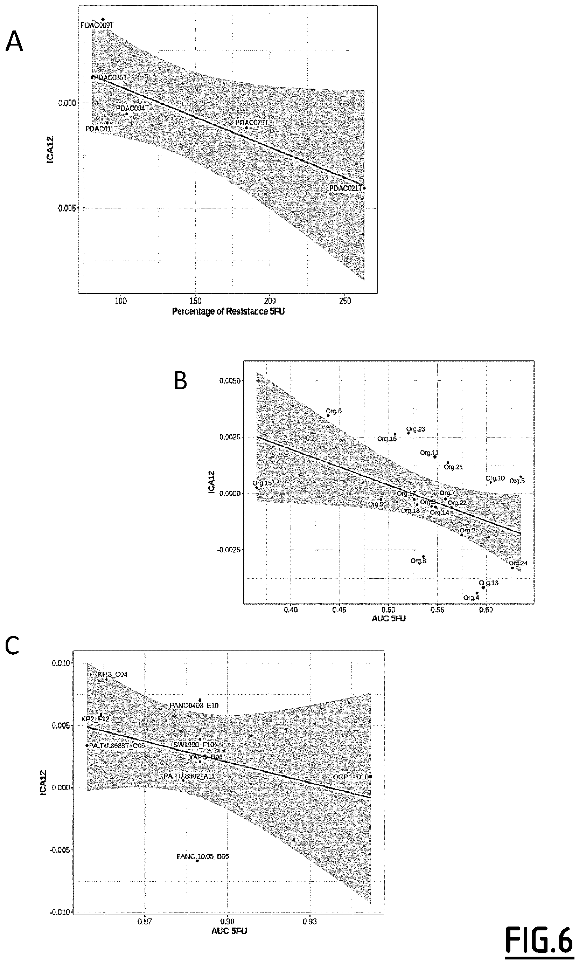

5-FU chemo-sensitivity signature (ICA12) correlates negatively with the AUC (Figure 5A; r = -0.50; P = 0.03; Table 3). Validation was performed in an independent PDPCC cohort (Figure 5B; r = -0.4; P = 0.03) and PDX (Figure 6A; r = -0.89; P = 0.02). Additionally, ICA12 predicted accurately BDPO (Figure 6B; r = -0.4; P = 0.04) and CACL (Figure 6C; r = -0.4; P = 0.03) chemo-sensitivity response.

### Irinotecan chemo-sensitivity signature

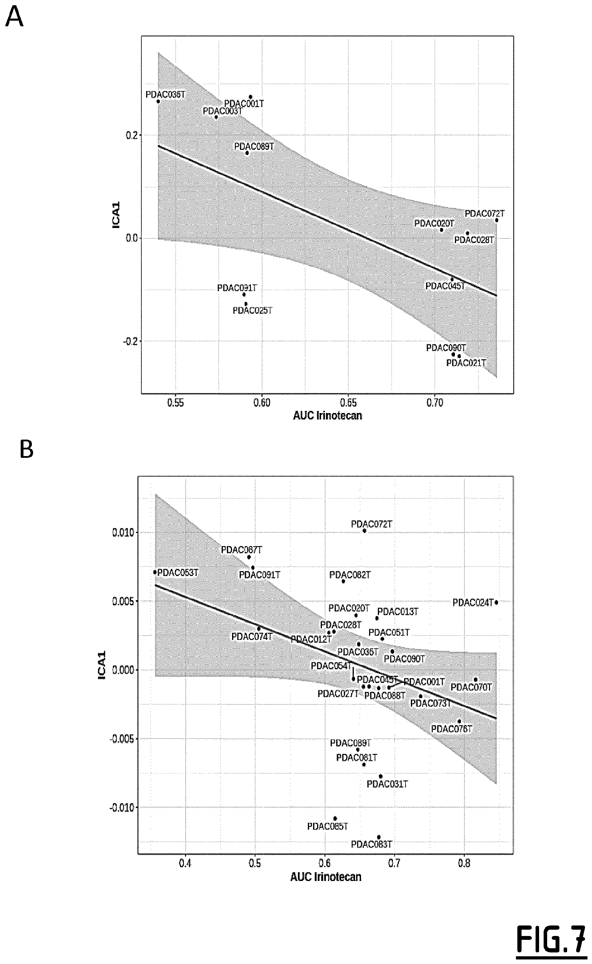

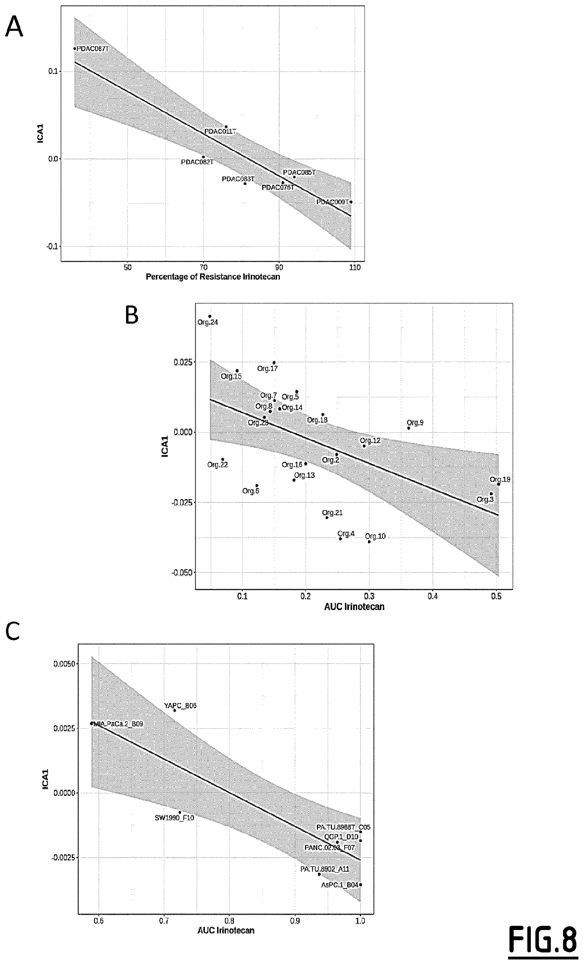

ICA1 component displayed a strong correlation between PDPCC and AUC (Figure 7A; r = 0.59; P = 0.04; Table 4). ICA1 component was validated in an independent cohort of PDPCC (Figure 7B; r = -0.43; P = 0.02) as well as an PDX cohort (Figure 8A; r = -0.82; P = 0.02). In addition, ICA1 was effective in predict BDPO (Figure 8B; r = -0.57; P = 0.007) and CACL (Figure 8C; r = -0.85; P = 0.007) chemo-sensitivity profile.

### Taxanes chemo-sensitivity signature

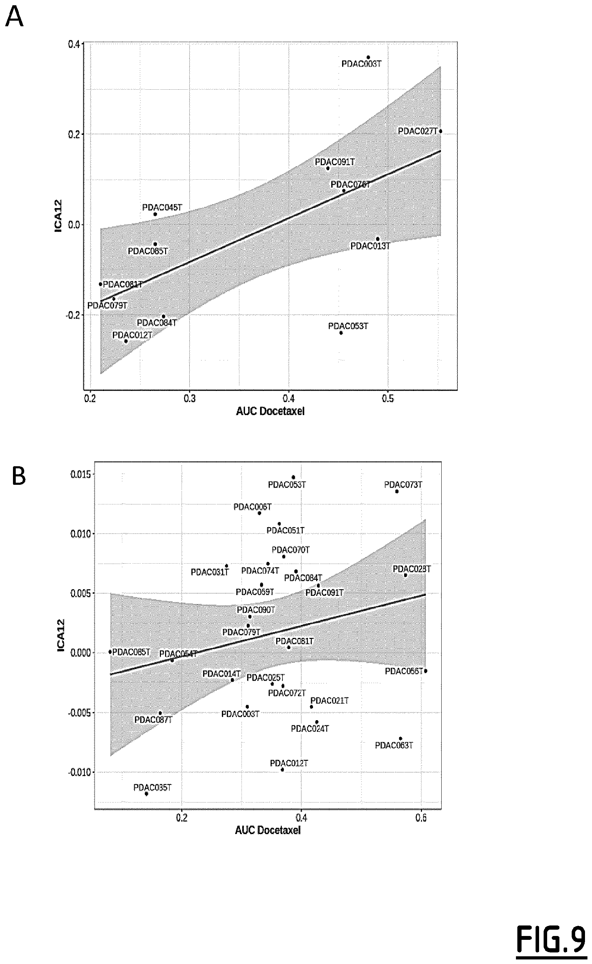

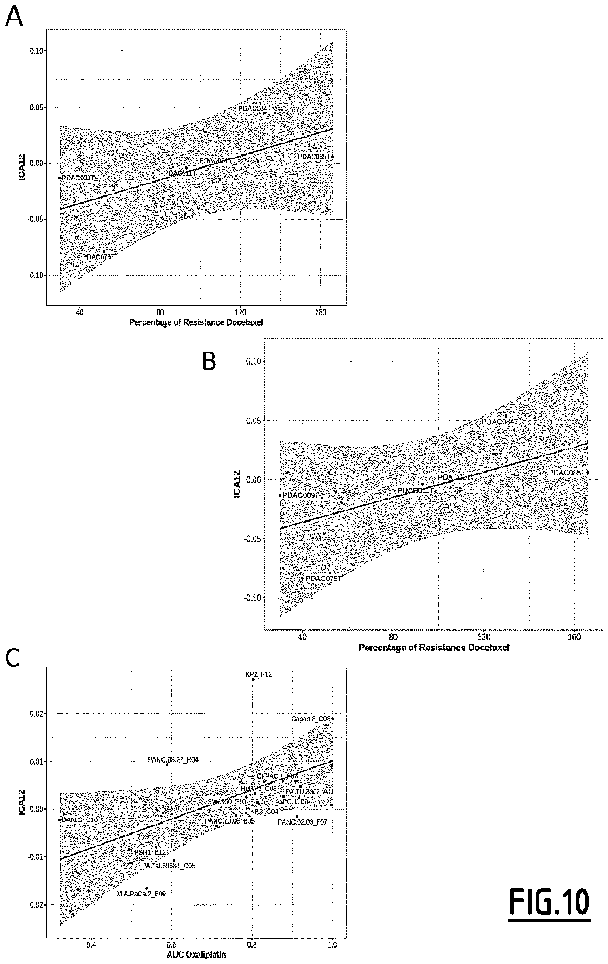

Docetaxel PDPCC AUC displayed a strong correlation with ICA12 component (Figure 9A; r = 0.64; P = 0.03). In addition, the selected component was validated in an independent cohort of PDPCC (Figure 9B; r = 0.41; P = 0.03) and PDX (Figure 10A; r = 0.89; P = 0.02). Docetaxel signature was projected in both, BDPO (Figure 10B; r = 0.46; P = 0.04) and CACL (Figure 10C; r = 0.52; P = 0.04), showing a high predictive power.

Conclusion: Since patients with a PDAC have a short survival time, and because when the disease progresses, the chances of response is decreasing, it is essential to give the best-adapted treatment to each patient as a first line. On the one hand, the chances of a response will be increased and, on the other hand, the toxic effects of ineffective drugs will be avoided.

## Claims

1. An *ex vivo* method for determining if a patient having pancreatic ductal adenocarcinoma (PDAC) is sensitive to a treatment, comprising:
a) detecting total RNA transcripts of cells of the PDAC of said patient; and
b) determining the patient as sensitive to said treatment, if the RNA signature of PDAC cells of said patient corresponds to the sensitivity RNA signature of said treatment,
wherein the sensitivity RNA signature of a treatment is a set of RNA transcripts which presence or absence is significantly correlated to sensitivity to said treatment.

2. An *ex vivo* method according to claim 1, for determining if the patient is sensitive to a treatment with gemcitabine, oxaliplatin, 5 fluoro-uracil (5-FU), irinotecan, a taxane or a combination thereof, wherein:
a) the patient is determined as being sensitive to Gemcitabine, if the RNA signature of PDAC cells corresponds to Table 1, and/or
b) the patient is determined as being sensitive to Oxaliplatin, if the RNA signature of PDAC cells corresponds to Table 2, and /or
c) the patient is determined as being sensitive to 5-FU, if the RNA signature of PDAC cells corresponds to Table 3, and/or
d) the patient is determined as being sensitive to Irinotecan, if the RNA signature of PDAC cells corresponds to Table 4, and/or
e) the patient is determined as being sensitive to taxane, if the RNA signature of PDAC cells corresponds to Table 5.

3. The method according to claim 2, wherein said taxane is docetaxel, paclitaxel or nab-paclitaxel.

4. The method according to claim 2, wherein if said patent is determined as sensitive to oxaliplatin, 5-FU and/or irinotecan said patient is thus sensitive to the FOLFIRINOX treatment.

5. The method according to any one of claims 1-4, wherein total RNA transcripts are detected by DNA microarray, RNA-seq, nanostring or RT-PCR.

6. The method according to any one of claims 1-5, wherein RNA transcripts of the sensitivity RNA signature of a treatment are RNA transcript of genes of table 1, and/or of table 2, and/or of table 3, and/or of table 4 and/or of table 5.

7. Gemcitabine, Oxaliplatin, 5 fluoro-uracil (5-FU), Irinotecan, a taxane or a combination thereof, for use for treating PDAC in a patient, wherein said patient having pancreatic ductal adenocarcinoma has been determined sensitive to gemcitabine, oxaliplatin, 5 fluoro-uracil (5-FU), irinotecan, a taxane or a combination thereof.

8. A kit for determining if a patient having pancreatic ductal adenocarcinoma (PDAC) is sensitive to a treatment with gemcitabine, oxaliplatin, 5 fluoro-uracil (5-FU), irinotecan, a taxane or a combination thereof, comprising means for detecting RNA of genes of tables 1, 2, 3, 4 or 5.

9. An *ex vivo* method for determining a suitable treatment for a patient having pancreatic ductal adenocarcinoma (PDAC), comprising:
a) determining if the patient is sensitive to a treatment using the method of any one of claims 1- 6, and
b) deducing a suitable treatment for the patient.

10. An *ex vivo* method according to claim 9, wherein step a) comprises determining if the patient is sensitive to a treatment with gemcitabine, oxaliplatin, 5 fluoro-uracil (5-FU), irinotecan, a taxane or a combination thereof using the method of any one of claims 2- 6.
